# FASCICULE DE BREVET EUROPEEN

(11) **EP 1 697 310 B1**
(45) Date de publication et mention de la délivrance du brevet: **16.07.2008**
(21) Numéro de dépôt: 04816421.4
(22) Date de dépôt: 17.12.2004
(51) Int. Cl.: C07C 241/02

(54) **PROCEDE DE SYNTHESE EN CONTINU DE MONOALKYL-HYDRAZINES A GROUPE ALKYLE FONCTIONNALISE**
VERFAHREN ZUR KONTINUIERLICHEN SYNTHESE VON MONOALKYLHYDRAZINEN MIT EINER FUNKTIONALISIERTEN ALKYLGRUPPE
METHOD FOR THE CONTINUOUS SYNTHESIS OF MONOALKYL-HYDRAZINES WITH A FUNCTIONALISED ALKYL GROUP

(30) Priorité: 17.12.2003 FR 0314796
(43) Date de publication de la demande: 06.09.2006
(73) Titulaire: ISOCHEM, 75004 Paris (FR); CENTRE NATIONAL DE LA RECHERCHE SCIENTIFIQUE (CNRS) Etablissement Public, 75016 Paris (FR); UNIVERSITE CLAUDE BERNARD - LYON 1, 69100 Villeurbanne (FR)
(72) Inventeur: DELALU, Henri, F-69002 Lyon (FR); COLAS-DURICHE, Cécile, F-33400 Talence (FR); BERTHET, Jacques, F-69003 Lyon (FR); LEURENT, Philippe, F-31400 Toulouse (FR)
(74) Mandataire: Ahner, Francis
(86) Numéro de dépôt international: PCT/FR2004/003286
(87) Numéro de publication internationale: WO 2005/058801

(56) Documents cités:
- EP-A- 0 462 016
- FR-A- 2 651 776
- GB-A- 1 095 040
- L.H. DIAMOND AND L.F. AUDRIETH: "Preparation of N-Substituted Hydrazines from Amines and Chloramine" J. AM. CHEM. SOC., vol. 77, 1955, page 3131, XP002324928

## Description

La présente invention concerne un procédé de synthèse en continu de monoalkyl-hydrazines à groupe alkyle fonctionnalisé.

Au sens de la présente invention, on entend par «monoalkyl-hydrazine» toute hydrazine de formule NH₂-NH-R dans laquelle R représente un groupe alkyle fonctionnalisé, c'est-à-dire comprenant au moins une fonction choisie dans le groupe constitué par une insaturation carbone-carbone (cas de l'allyle hydrazine) ou carbone-azote, un groupe alkoxy (cas de la 2-méthoxyethyl hydrazine) ou phénoxy (cas de Ph-O-CH₂-CH₂ NH-NH₂), une fonction amine tertiaire (cas de (Me)₂N-CH₂-CH₂-NH-NH₂) ou un groupe phényle (cas de Ph- CH₂- NH-NH₂).

Les monoalkyl-hydrazines, notamment l'allylhydrazine, sont des composés fréquemment utilisés comme intermédiaires dans la fabrication de médicaments.

A l'heure actuelle, les seules méthodes de synthèse décrites dans la littérature scientifique font appel à l'hydrate d'hydrazine N₂H₄ et aux nitrosamines. Dans le cas particulier de la synthèse de l'allylhydrazine, la première méthode consiste à ajouter progressivement 1,28 moles de bromure d'allyle dans 8,96 moles de monohydrate d'hydrazine, ce qui correspond à un rapport molaire de 7. Au cours de l'addition, la température doit être maintenue inférieure à 40°C. Le mélange réactionnel est ensuite chauffé sous reflux à 70°C pendant une heure. Après l'extraction à l'éther et distillation, on obtient un mélange constitué de 57% de monoallylhydrazine (CH₂=CHCH₂NHNH₂), 11% de diallylhydrazine ((CH₂=CHCH₂)₂NNH₂) et de triallylhydrazine La mise en oeuvre d'un rapport plus élevé diminue la quantité de monoallylhydrazine au profit de la diallylhydrazine (rendement en (CH₂=CHCH₂)₂NNH₂ de 34,6%). Les difficultés de l'allylation se situent au niveau de la non sélectivité et de la séparation de l'allylhydrazine dans les mélanges mono-, di-, tri-allylhydrazine / eau/ N₂H₄ (loffe B.V. et al. Zh. Org. Khim (1967) 3(6), 938-8). Une série de brevets (JP 93-256100 ; JP 93-261194 ; JP 7118218 ; JP 7112963) faisant appel à différentes méthodes ont été déposés pour parvenir in fine à un composé de haute pureté.

Une seconde méthode de synthèse de l'allylhydrazine consiste en une nitrosation de l'allylamine à basse température (5°C) suivie d'une hydrogénation chimique (LiAlH₄) du dérivé nitrosé (1-nitrosoallylamine) en milieu éthéré. Le rendement réactionnel ne dépasse pas 42%. Cependant, le produit issu de la première étape doit être manipulé avec beaucoup de précautions à cause de sa toxicité (composé hautement cancérogène), ce qui pose industriellement des problèmes de mise en exploitation. De plus, l'utilisation de LiAlH₄ impose l'absence de traces d'eau, des réacteurs étanches et des solvants anhydres (éther diéthylique), ce qui a pour effet d'augmenter les risques d'inflammation du mélange réactionnel.

D'autre part, il est reconnu que pour la préparation de différentes hydrazines, on peut faire appel à la réaction dite de « Raschig », qui consiste à synthétiser la monochloramine par réaction de l'ammoniac sur une solution d'hypochlorite de sodium et à faire réagir la monochloramine formée sur une amine pour obtenir l'hydrazine correspondante. Ce procédé est assez difficile à mettre en oeuvre parce qu'il nécessite deux étapes distinctes, la première réalisée à froid pour la synthèse de la monochloramine et la deuxième réalisée à chaud, pendant laquelle est effectuée la synthèse de l'hydrazine. Par ailleurs, la monochloramine doit se trouver en présence d'un excès suffisant d'amine dans les solutions intermédiaires de manière à éviter des réactions secondaires de dégradation, et par la suite le procédé exige toujours des quantités très importants de solutions à traiter. Cependant, ce procédé ne peut pas être appliqué pour la préparation de toutes les alkyl-hydrazines et surtout pas pour la préparation d'alkyl-hydrazines monosubstituées. En plus, le traitement des solutions de synthèse nécessite l'extraction de l'eau puis de l'amine, ce qui exige des opérations onéreuses.

Les inventeurs ont maintenant découvert un nouveau procédé de synthèse de monoalkyl-hydrazines, en particulier de l'allylhydrazine. Ce procédé mis en oeuvre en continu est basé sur une transposition du procédé Raschig, et il consiste à préparer la chloramine par action de l'hypochlorite de sodium sur l'ammoniac à basse température, et ensuite, à faire agir la chloramine ainsi produite sur l'alkyl-amine en milieu homogène ou hétérogène, puis à recycler l'amine et à extraire l'hydrazine formée.

L'amine de départ peut être recyclée.

L'invention permet de disposer d'un procédé simple et économique pour l'obtention d' allcyl-hydrazines.

La présente invention a donc pour objet un procédé de synthèse en continu d'une monoalkyl-hydrazine de formule (I)

NH₂-NH-R

dans laquelle R représente indépendamment un radical alcényle en C₂-C₆, un radical alcynyle en C₂-C₆, un radical alkyle linéaire en C₁-C₅ contenant au moins une fonction imino (-C=N-) ou un radical alkyle en C₁-C₆ linéaire ou ramifié portant au moins un groupement fonctionnel choisi dans le groupe constitué par les radicaux alkoxy en C₁-C₆, C=NH, C≡N, phénoxy, COO-alkyle en C₁-C₆, phényle ou NR₃R₄, R₃ et R₄ représentant indépendamment l'un de l'autre un radical alkyle en C₁-C₆ ou formant un cycle en C₂-C₆ ; caractérisé en ce qu'il comprend les étapes successives suivantes :
a) synthétiser la monoallcyl-hydrazine de formule (I) dans un réacteur approprié en faisant réagir en milieu alcalin et à une température comprise entre 25 et 45°C une monochloramine avec une amine anhydre de formule NH₂-R (II), R ayant la même signification que pour la formule (I) ; puis
b) démixter la solution obtenue suite à l'étape a) en une phase organique et une phase aqueuse par l'ajout d'hydroxyde de sodium anhydre sous refroidissement afin que la température du milieu de démixtion ne dépasse pas les températures d'ébullition des composés ; et
c) isoler à partir de la phase organique ainsi obtenue la monoalkyl-hydrazine de formule (I).

Lors de l'étape a), la monochloramine et l'amine anhydre de formule (II) sont avantageusement introduites de manière simultanée.

La synthèse de la monoaikyl-hydrazine de formule 1 dans l'étape a) est effectuée en milieu homogène ou en milieu hétérogène dans un réacteur approprié, qui est avantageusement un réacteur tubulaire agité. Le réacteur tubulaire permet d'éviter un contact entre la monoalkyl-hydrazine naissante et la monochloramine et ainsi il permet d'éviter une réaction d'oxydo-réduction entre ces deux réactifs. Le front réactionnel se déplace le long du tube et la monoalkyl-hydrazine n'est plus en contact avec la monochloramine injectée à la base du réacteur.

Selon une variante avantageuse de l'invention, la concentration en ions hydroxyles dans le milieu de réaction de l'étape a) est comprise entre 0,3 et 0,8 mol.l⁻¹.

Selon une variante avantageuse de l'invention, à l'étape a), le rapport molaire amine anhydre de formule II/monochloramine est compris entre 18 et 30, les bornes étant incluses. Le temps de réaction est variable est dépend de la température à laquelle s'effectue la réaction et du rapport des concentrations des réactifs. Par exemple, dans le cas de la synthèse de la monoallyhydrazine, dans la gamme des rapports de concentrations donnée et à 25°C, le temps de réaction est de l'ordre de 2 à 10 minutes.

Selon une variante avantageuse de l'invention, préalablement à l'étape a) la monochloramine est alcalinisée dans un mélangeur par ajout d'une solution d'hydroxyde de sodium de telle sorte que le titre massique en hydroxyde de sodium dans le mélangeur soit compris entre 2 et 6%. Le mélangeur est avantageusement maintenu à une température comprise entre -10 et 5°C.

Ainsi, selon cette variante de l'invention, la réaction de la monochloramine avec l'amine anhydre de formule (II) s'effectue en présence d'une solution aqueuse d'hydroxyde de sodium à une température comprise entre 25 et 45°C. A la sortie du réacteur de l'étape a), c'est-à-dire en fin de réaction, la concentration en hydroxyde de sodium est inférieure à 0,3 mol/L. La concentration en soude ne doit pas être trop élevée sinon le mélange réactionnel risque de démixter par relargage. En cas de relargage, il faudrait alors faire intervenir un réacteur du type réacteur piston agité.

Lors de la réaction de synthèse de la monoalkyl-hydrazine, de l'acide chlorhydrique est également formé. L'alcalinisation de la monochloramine, c'est-à-dire l'ajout d'une base forte telle que la soude, permet de neutraliser l'acide formé, afin d'éviter toute protonation locale de l'amine au moment du mélange et ainsi éviter la formation d'une monochloramine substituée, qui pourrait engendrer la formation d'alkyl-hydrazines di- ou tri-substituées. La quantité de base forte ajoutée doit être suffisante pour neutraliser tout l'acide formé. De plus, la vitesse de formation de l'hydrazine augmente avec l'alcalinité du milieu, ce qui n'est pas le cas des réactions de dégradation, telles que par exemple l'oxydation de l'hydrazine naissante par la chloramine.

La monochloramine, avantageusement alcalinisée, et l'amine anhydre de formule (II) sont de manière avantageuse introduites simultanément dans le réacteur. Les débits d'addition de l'amine anhydre de formule (II) et de la monochloramine sont tels que le rapport des concentrations molaires de l'amine hétérocyclique sur la monochloramine soit avantageusement compris entre 18 et 30, les bornes pouvant être incluses. Dans le cas de la synthèse de la monoallylhydrazine, la réaction de synthèse s'effectue en milieu homogène.

Lors de l'étape b), la quantité d'hydroxyde de sodium anhydre ajoutée est avantageusement telle que le titre massique en hydroxyde de sodium soit compris entre 10 et 35%, de préférence de 30%. Dans ces conditions, le milieu démixte en deux phases dont l'une concentre la quasi-totalité des molécules organiques, notamment la monoalkyl-hydrazine et l'amine initiale, dans la phase légère (phase organique). Ce traitement à l'hydroxyde de sodium permet par démixtion d'éliminer au moins 70 à 80% en poids, selon le caractère organique (nombre d'atomes de carbones) de l'amine et de la monoalkylhydrazine, avantageusement environ 85% en poids de l'eau présente dans le milieu réactionnel et d'extraire l'ammoniac formé avec les sels dans la phase inférieure (phase aqueuse). Le taux d'eau diminue avec le nombre d'atomes de carbones ou au contraire augmente s'il y a des groupes fonctionnels hydrophiles.

Dans le cas de la synthèse de l'allylhydrazine par exemple, la température du milieu de démixtion de l'étape b) ne doit pas dépasser 80°C.

L'étape c) comprend avantageusement les étapes successives suivantes :
i) isoler l'amine anhydre de formule (II) qui n'a pas réagi et une solution concentrée de monoalkyl-hydrazine de formule (I) par distillation de la phase organique obtenue suite à l'étape b) ; puis
ii) le cas échéant, purifier ladite solution concentrée de monoalkyl-hydrazine de formule I.

La distillation, avantageusement conduite à pression atmosphérique, permet de récupérer en tête de colonne la totalité de l'amine anhydre de formule (II) initiale qui n'a pas réagi à une température de distillation égale ou légèrement supérieure à la température d'ébullition de ladite amine sans entraînement de la monoalkyl-hydrazine formée qui a une température d'ébullition plus élevée.

Ladite amine récupérée suite à l'étape i) est avantageusement réinjectée dans le réacteur de l'étape a). Ladite amine peut être réinjectée directement, sans traitement supplémentaire, au niveau du réacteur de l'étape a) où se forme la monoalkyl-hydrazine. La solution concentrée de monoalkyl-hydrazine de formule (I) est le cas échéant purifiée, avantageusement par distillation, qui peut être effectuée à pression atmosphérique. Cette distillation, appelée rectification finale, permet d'obtenir en tête de colonne un titre supérieur à 95%, avantageusement supérieur à 99%, en monoalkyl-hydrazine. Ladite distillation est éventuellement précédée d'une étape de démixtion en une phase organique et une phase aqueuse par ajout d'hydroxyde de sodium anhydre de telle sorte que le titre massique en hydroxyde de sodium soit compris entre 30 et 50%. Cette étape de démixtion permet d'éliminer l'eau éventuellement encore présente dans la solution concentrée de monoalkyl-hydrazine obtenue suite à l'étape i).

La monochloramine introduite à l'étape a) est avantageusement préparée selon un procédé comprenant les étapes successives suivantes :
α) préparer une solution aqueuse d'hypochlorite de sodium ayant un degré chlorométrique compris entre 36 et 100° éventuellement par dilution d'une solution d'hypochlorite ayant un degré chlorométrique compris entre 100 et 120° ; puis
β) faire réagir une solution d'hydroxyde d'ammonium et de chlorure d'ammonium avec la solution aqueuse d'hypochlorite de sodium obtenue suite à l'étape α), en milieu faiblement alcalin, à une température comprise entre -15 et -7°C, pour former ladite monochloramine.

Au sens de la présente invention, on entend par l'expression milieu « faiblement alcalin » un milieu dont la valeur de pH est d'environ 10±1.

Le rapport molaire solution d'hydroxyde d'ammonium et de chloruré d'ammonium/solution aqueuse d'hypochlorite de sodium est avantageusement compris entre 2,5 et 3, les bornes étant incluses.

Le rapport molaire chlorure d'ammonium/hydroxyde d'ammonium est avantageusement compris entre 0,1 et 1,75, les bornes étant incluses, plus avantageusement il est d'environ 0,65.

Dans le cas où le réactif chloré mis en oeuvre à l'étape α) est obtenu par dilution d'une solution d'hypochlorite haut titre à 100-120°chlorométrique, cette dilution présente l'avantage de diminuer de 40% la teneur en chlorure de sodium. Ce traitement, favorable pour l'environnement, permet un refroidissement de la solution d'eau de Javel, sans risque de cristallisation jusqu'à -15°C.

Le procédé mis au point, objet de la présente découverte, permet d'obtenir une parfaite sélectivité au niveau de la monoalkyl-hydrazine sans la présence de ses formes di- et tri-substituées, ce qui est une des originalités majeures par rapport aux procédés faisant intervenir une alkylation. On peut considérer que le procédé de synthèse selon l'invention ne conduit jamais à des produits di- ou tri- substitués. En effet, le réactif aminant non substitué, en l'occurrence la monochloramine, cède son groupe NH₂- à l'amine de formule (II) par substitution nucléophile de type SN2. L'alkyl-hydrazine monosubstituée résultante conservera donc le même degré de substitution que l'amine précurseur. Ce procédé simple à mettre en oeuvre évite les différents traitements antérieurs complexes d'isolement de la monoalkyl-hydrazine en présence de mélange H₂O-N₂H₄.

Le procédé de la présente invention permet donc non seulement la synthèse de la monoalkyl-hydrazine en continu, sans formation d'aucun intermédiaire toxique, mais il permet aussi l'obtention de ladite hydrazine avec un coût peu élevé.

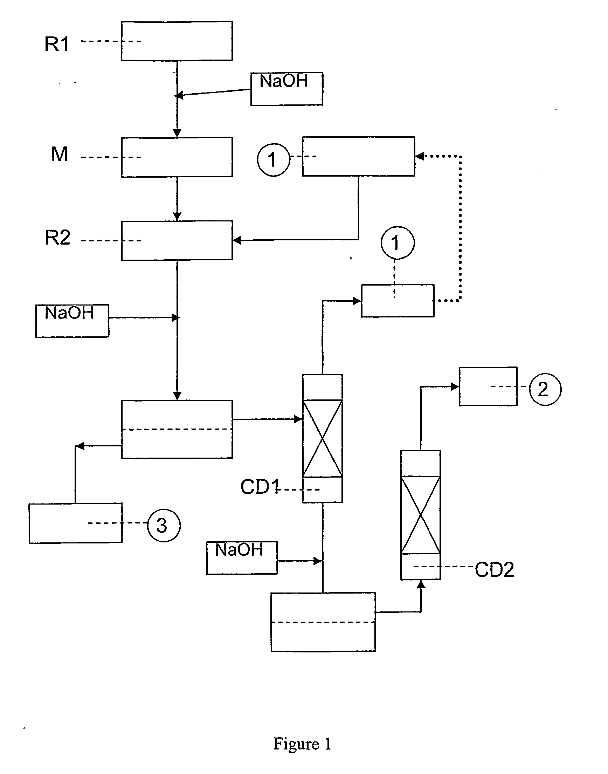

L'exemple donne, à titre non limitatif, une description détaillée de la mise en oeuvre du procédé de l'invention, procédé dont le schéma de principe est représenté figure 1.

Signification des abréviations utilisées :
- R1 :: réacteur 1
- M :: mélangeur
- R2 :: réacteur 2
- CD1 :: colonne de distillation n°1
- CD2 :: colonne de distillation n°2
- 1 :: allylamine anhydre
- 2 :: allylhydrazine
- 3 :: Solution eau+NH₃+NaCl+NaOH

### EXEMPLE : Préparation de la monoallylhydrazine en continu

Toutes les quantités indiquées correspondent à une unité en régime et sont rapportées à un litre d'hypochlorite injecté.

Un litre d'une solution d'hypochlorite de sodium élaborée par dilution de 50% d'une solution d'hypochlorite de haut titre (100 à 120° chlorométrique, soit après dilution [NaOCl] = 2,14 mol.L⁻¹ ; [NaCl] = 0,85 mol.L⁻¹) et un litre de solution ayant une concentration en ammoniac de 3,60 mol.L⁻¹ et en chlorure d'ammonium de 2,38 mol.L⁻¹ sont introduits en continu dans un réacteur agité (RI) à raison de 5 mL.min⁻¹ chacun (soit 6 g/min de solution d'hypochlorite à 48°chlorométrique et 5,05 g/min du mélange ammoniacal NH₃ + NH₄Cl).

La température au sein du réacteur est maintenue entre -8°C et -11°C, et le pH de la réaction est voisin de 10. A la sortie de R1, on obtient une solution de monochloramine de titre supérieur à 1 mol.L⁻¹, ce qui correspond à un rendement proche de 100% par rapport à l'hypochlorite de sodium.

A la sortie de R1, la solution de monochloramine obtenue ci-dessus (2 litres) est alcalinisée par introduction en continu d'une solution concentrée d'hydroxyde de sodium (0,39 litre à 30% en poids) au sein d'un mélangeur M à double enveloppe maintenu à basse température entre -9°C et -11°C. L'homogénéisation est assurée par entraînement magnétique.

La synthèse de la monoallylhydrazine est effectuée au moyen d'un agitateur tubulaire R2 agité. La monochloramine alcalinisée (2,39 litres), issue de l'enceinte de mélange M, et l'allylamine anhydre (3,25 litres soit 2,46 kg car la densité est de 0,760) sont introduits simultanément à la base du réacteur au moyen de pompes doseuses. Le débit de l'allylamine anhydre est de 16,46 mL/min et une partie de la réaction est effectuée en milieu homogène à 35°C. La concentration finale en NaOH au sortir de R2 est de 0,3 mol.L⁻¹.

Le présent procédé est caractérisé en ce que l'on ajoute, à la liqueur réactionnelle homogène (5,6 kg), de l'hydroxyde de sodium, selon un titre massique compris de préférence entre 30 et 40%, sous refroidissement de manière que la température ne dépasse pas 45°C. Dans ces conditions, on obtient deux phases dont l'une légère (environ 2 kg) contient la totalité des organiques, c'est à dire la monoallylhydrazine et l'allylamine en excès. Ce traitement permet ainsi d'éliminer entre 80 et 85% en poids de l'eau présente dans les solutions de synthèse.

L'obtention de la monoallhylhydrazine nécessite ensuite deux étapes successives :
- récupération de l'allylamine qui n'a pas réagi par distillation de la phase à la pression atmosphérique. On récupère environ 1,7 kg d'amine anhydre à une température de 52°C (colonne de distillation CD1), qui est réinjecté sans traitement dans le réacteur R2.
- Purification de la solution obtenue en pied de colonne (colonne de distillation CD2) après séparation par addition d'hydroxyde de sodium (40 à 50%). Avant purification, la phase légère a un titre en monoallhylhydrazine au moins égal à 95%.

Après purification, on obtient de la monoallhylhydrazine ayant un degré de pureté supérieur à 99%. Le rendement en monoallylhydrazine par rapport à l'allylamine consommée est supérieur à 80%.

## Revendications

1. Procédé de synthèse en continu d'une monoalkyl-hydrazine de formule (I)
NH₂-NH-R
dans laquelle R représente indépendamment un radical alcényle en C₂-C₆, un radical alcynyle en C₂-C₆ un radical alkyle en C₁-C₅ contenant une fonction imino (-C=N-) ou un radical alkyle en C₁-C₆ linéaire ou ramifié portant au moins un groupement fonctionnel choisi dans le groupe constitué par les radicaux alkoxy en C₁-C₆, C=NH, C≡N, phénoxy, COO-alkyle en C₁-C₆, phényle ou NR₃R₄, R₃ et R₄ représentant indépendamment l'un de l'autre un radical alkyle en C₁-C₆ ou formant un cycle en C₂-C₆ ; **caractérisé en ce qu'**il comprend les étapes successives suivantes :
a) synthétiser la monoalkyl-hydrazine de formule (I) dans un réacteur approprié en faisant réagir en milieu alcalin et à une température comprise entre 25 et 45°C une monochloramine avec une amine anhydre de formule NH₂-R (formule (II)), R ayant la même signification que pour la formule (I) ; puis
b) démixter la solution obtenue suite à l'étape a) en une phase organique et une phase aqueuse par l'ajout d'hydroxyde de sodium anhydre sous refroidissement afin que la température du milieu de démixtion ne dépasse pas les températures d'ébullition des composés ; et
c) isoler à partir de la phase organique ainsi obtenue la monoalkyl-hydrazine de formule (I).

2. Procédé selon la revendication 1, **caractérisé en ce que**, à l'étape a), le rapport molaire amine anhydre de formule II/monochloramine est compris entre 18 et 30.

3. Procédé selon la revendication 1 ou 2, **caractérisé en ce que** le réacteur approprié utilisé à l'étape a) est un réacteur tubulaire agité.

4. Procédé selon l'une quelconque des revendications précédentes, **caractérisé en ce que** préalablement à l'étape a) la monochloramine est alcalinisée dans un mélangeur par ajout d'une solution d'hydroxyde de sodium de telle sorte que le titre massique en hydroxyde de sodium soit compris entre 2 et 6%.

5. Procédé selon la revendication 4, **caractérisé en ce que** le mélangeur est maintenu à une température comprise entre -10 et 5°C.

6. Procédé selon l'une quelconque des revendications précédentes, **caractérisé en ce que** la quantité d'hydroxyde de sodium anhydre ajoutée lors de l'étape b) est telle que le titre massique en hydroxyde de sodium soit compris entre 10 et 35%.

7. Procédé selon l'une quelconque des revendications précédentes, **caractérisé en ce que** l'étape c) comprend les étapes successives suivantes :
i) isoler l'amine anhydre de formule (II) qui n'a pas réagi et une solution concentrée de monoalkyl-hydrazine de formule (I) par distillation de la phase organique obtenue suite à l'étape b) ; puis
ü) le cas échéant, purifier ladite solution concentrée de monoalkyl-hydrazine de formule (I).

8. Procédé selon la revendication 7, **caractérisé en ce que** ladite amine anhydre de formule (II) qui n'a pas réagi récupérée suite à l'étape i) est réinjectée dans le réacteur de l'étape a).

9. Procédé selon la revendication 7, **caractérisé en ce que** la solution concentrée de monoalkyl-hydrazine de formule (I) est purifiée par distillation, ladite distillation étant éventuellement précédée d'une étape de démixtion en une phase organique et une phase aqueuse par ajout d'hydroxyde de sodium anhydre de telle sorte que le titre massique en hydroxyde de sodium soit compris entre 30 et 50%.

10. Procédé selon l'une quelconque des revendications précédentes, **caractérisé en ce que** la monochloramine est préparée selon un procédé comprenant les étapes successives suivantes :
α) préparer une solution aqueuse d'hypochlorite de sodium ayant un degré chlorométrique compris entre 36 et 100° éventuellement par dilution d'une solution d'hypochlorite ayant un degré chlorométrique compris entre 100 et 120° ; puis
β) faire réagir une solution d'hydroxyde d'ammonium et de chlorure d'ammonium avec la solution aqueuse d'hypochlorite de sodium obtenue suite à l'étape a), en milieu faiblement alcalin, une température comprise entre -15 et -7°C, pour former ladite monochloramine.

11. Procédé selon la revendication 10, **caractérisé en ce que** le rapport molaire solution d'hydroxyde d'ammonium et de chlorure d'ammonium/solution aqueuse d'hypochlorite de sodium est compris entre 2,5 et 3.

12. Procédé selon la revendication 10 ou 11, **caractérisé en ce que** le rapport molaire chlorure d'ammonium/hydroxyde d'ammonium est compris entre 0,1 et 1,75, avantageusement il est d'environ 0,65.

## Claims

1. A method for the continuous synthesis of a monoalkylhydrazine of formula (I)
NH₂-NH-R
in which R represents independently an alkenyl radical at C₂-C₆, an alkynyl radical at C₂-C₆, a linear alkyl radical at C₁-C₅ containing at least one imine function (-C=N-) or a linear or branched alkyl radical at C₁-C₆ carrying at least one functional group selected from the group comprised of the radicals alkoxy at C₁-C₆, C=NH, C≡N, phenoxy, COO-alkyl at C₁-C₆, phenyl or NR₃R₄, R₃ and R₄ each representing independently an alkyl radical at C₁-C₆ or forming a ring at C₂-C₆, **characterized in that** it comprises the following successive steps:
a) synthesizing the monoalkylhydrazine of formula (I) in a suitable reactor while causing to react in an alkaline medium and at a temperature in the range between 25 and 45 °C a monochloramine with an anhydrous amine of formula NH₂-R (formula (II)), R having the same significance as for formula (I); then
b) demixing the solution obtained following step a) in an organic phase and an aqueous phase by the addition of anhydrous sodium hydroxide under cooling so that the temperature of the demixing medium does not exceed the boiling points of the compounds; and
c) isolating from the organic phase thus obtained the monoalkylhydrazine of formula (I).

2. The method according to claim 1, **characterized in that**, in step a), the formula II anhydrous amine/monochloramine molar ratio is in the range between 18 and 30.

3. The method according to either of claims 1 or 2, **characterized in that** the appropriate reactor to use in step a) is a stirred tubular reactor.

4. The method according to any of the preceding claims, **characterized in that** before step a) the monochloramine is alkalized in a mixer by the addition of a solution of sodium hydroxide in such a way that the weight concentration in sodium hydroxide is in the range between 2% and 6%.

5. The method according to claim 4, **characterized in that** the mixer is maintained at a temperature in the range between -10 and 5°C.

6. The method according to any of the preceding claims, **characterized in that** the quantity of the anhydrous sodium hydroxide added during step b) is such that the weight concentration in sodium hydroxide is in the range between 10% and 35%.

7. The method according to any of the preceding claims, **characterized in that** step c) comprises the following successive steps:
i) isolating the unreacted anhydrous amine of formula (II) and a concentrated solution of the monoalkylhydrazine of formula (I) by distillation of the organic phase obtained following step b); then
ii) if necessary, purifying the aforesaid concentrated solution of the monoalkylhydrazine of formula (I).

8. The method according to claim 7, **characterized in that** the aforesaid unreacted anhydrous amine of formula (II) recovered following step i) is reinjected into the reactor of step a) .

9. The method according to claim 7, **characterized in that** the concentrated monoalkylhydrazine solution of formula (I) is purified by distillation, said distillation being possibly preceded by a step of demixing into an organic phase and an aqueous phase by the addition of anhydrous sodium hydroxide in such a way that the weight concentration in sodium hydroxide is in the range between 30% and 50%.

10. The method according to any of the preceding claims, **characterized in that** the monochloramine is prepared according to a method comprising the successive following steps:
a) preparing an aqueous sodium hypochlorite solution having a chlorometric degree in the range between 36 and 100°, possibly by the dilution of a hypochlorite solution having a chlorometric degree in the range between 100 and 120°; then
β) reacting a solution of ammonium hydroxide and of ammonium chloride with the aqueous sodium hypochlorite solution obtained following step α), in a slightly alkaline medium, at a temperature in the range between -15 and -7 °C, in order to form the said monochloramine.

11. The method according to claim 10, **characterized in that** the molar ratio of the ammonium hydroxide and ammonium chloride solution/aqueous sodium hypochlorite solution advantageously lies between 2.5 and 3.

12. The method according to claim 10 or 11, **characterized in that** the molar ratio of the ammonium chloride/ammonium hydroxide lies between 0.1 and 1.75, advantageously it is approximately 0.65.

## Patentansprüche

1. Verfahren zur kontinuierlichen Synthese eines Monoalkylhydrazins der Formel (I)
NH₂-NH-R
wobei R unabhängig ein C₂-C₆-Alkenylradikal, ein C₂-C₆-Alkynylradikal, ein C₁-C₅-Alkylradikal mit einer Imino-Funktion (-C=N-) oder ein lineares oder verzweigtes C₁-C₆-Alkylradikal, das mindestens eine funktionelle Gruppe trägt, die aus der Gruppe ausgewählt ist, die von den C₁-C₆-Alkoxyradikalen, C=NH, C=N, Phenoxyradikalen, C₁-C₆-COO-Alkylradikalen, Phenylradiakalen oder NR₃R₄ gebildet ist, wobei R₃ und R₄ unabhängig voneinander ein C₁-C₆-Alkylradikal darstellen oder einen C₂-C₆-Ring bilden, darstellt **dadurch gekennzeichnet, dass** es die folgenden aufeinanderfolgenden Schritte umfasst:
a) Synthetisieren des Monoalkylhydrazins der Formel (I) in einem geeigneten Reaktor durch Reaktion eines Monochloramins mit einem wasserfreien Amin der Formel NH₂-R (Formel (II)), wobei R dieselbe Bedeutung wie für die Formel (I) hat, in alkalischem Milieu und bei einer Temperatur zwischen 25 und 45 °C inklusive, danach
b) Entmischen der in Schritt a) hergestellten Lösung in eine organische Phase und eine wässrige Phase durch Hinzufügen von wasserfreiem Natriumhydroxid unter Abkühlung, damit die Temperatur des Entmischungsmilieus nicht die Siedetemperaturen der Verbindungen überschreitet, und
c) Isolieren des Monoalkylhydrazins der Formel (I) aus der hergestellten organischen Phase.

2. Verfahren nach Anspruch 1, **dadurch gekennzeichnet, dass** in Schritt a) das molare Verhältnis wasserfreies Amin der Formel II/Monochloramin zwischen 18 und 30 inklusive ist.

3. Verfahren nach Anspruch 1 oder 2, **dadurch gekennzeichnet, dass** der in Schritt a) verwendete geeignete Reaktor ein Röhren-Rühr-Reaktor ist.

4. Verfahren nach einem der vorangehenden Ansprüche, **dadurch gekennzeichnet, dass** das Monochloramin vor Schritt a) in einem Mischer durch Hinzufügen einer Natriumhydroxidlösung derart alkalisiert wird, dass Natriumhydroxid-Massegehalt zwischen 2 und 6 % inklusive ist.

5. Verfahren nach Anspruch 4, **dadurch gekennzeichnet, dass** der Mischer bei einer Temperatur zwischen -10 und 5 °C gehalten wird.

6. Verfahren nach einem der vorangehenden Ansprüche, **dadurch gekennzeichnet, dass** die bei Schritt b) hinzugefügte Menge wasserfreien Natriumhydroxids derart ist, dass der Natriumhydroxid-Massegehalt zwischen 10 und 35 % ist.

7. Verfahren nach einem der vorangehenden Ansprüche, **dadurch gekennzeichnet, dass** der Schritt c) die folgenden aufeinanderfolgenden Schritte umfasst:
i) Isolieren des wasserfreien Amins der Formel (II), das nicht reagiert hat, und einer konzentrierten Monoalkylhydrazinlösung der Formel (I) durch Destillation der in Schritt b) hergestellten organischen Phase, danach
ii) Reinigen der konzentrierten Monoalkylhydrazinlösung der Formel (I), sofern notwendig.

8. Verfahren nach Anspruch 7, **dadurch gekennzeichnet, dass** das wasserfreie Amin der Formel (II), das nicht reagiert hat und in Schritt i) wiedergewonnen wurde, wieder in den Reaktor von Schritt a) eingeführt wird.

9. Verfahren nach Anspruch 7, **dadurch gekennzeichnet, dass** die konzentrierte Monoalkylhydrazinlösung der Formel (I) durch Destillation gereinigt wird, wobei der Destillation eventuell ein Schritt des Entmischens in eine organische Phase und eine wässrige Phase durch Hinzufügen von wasserfreiem Natriumhydroxid vorausgeht derart, dass der Natriumhydroxid-Massegehalt zwischen 30 und 50 % inklusive ist.

10. Verfahren nach einem der vorangehenden Ansprüche, **dadurch gekennzeichnet, dass** das Monochloramin nach einem Verfahren zubereitet wird, dass die folgenden aufeinanderfolgenden Schritte umfasst:
α) Zubereitung einer wässrigen Natriumhypochloritlösung mit einem chlorometrischen Grad zwischen 36 und 100° inklusive, eventuell durch Verdünnen einer Hypochloritlösung mit einem chlorometrischen Grad zwischen 100 und 120 ° inklusive, danach
β) Reaktion einer Ammoniumhydroxid- und Ammoniumchloridlösung mit der in Schritt α) hergestellten wässrigen Natriumhypochloritlösung in einem schwach alkalischen Milieu bei einer Temperatur zwischen -15 und -7 °C inklusive, um das Monochloramin zu bilden.

11. Verfahren nach Anspruch 10, **dadurch gekennzeichnet, dass** das molare Verhältnis Ammoniumhydroxid- und Ammoniumchloridlösung/wässrige Natriumhypochloritlösung zwischen 2,5 und 3 inklusive ist.

12. Verfahren nach Anspruch 10 oder 11, **dadurch gekennzeichnet, dass** das molare Verhältnis Ammoniumchlorid/Ammoniumhydroxid zwischen 0,1 und 1,75 inklusive ist, in vorteilhafter Weise beträgt es zirka 0,65.
